# FASCICULE DE BREVET EUROPEEN

(11) **EP 2 130 821 B1**
(45) Date de publication et mention de la délivrance du brevet: **21.09.2011**
(21) Numéro de dépôt: 09162086.4
(22) Date de dépôt: 05.06.2009
(51) Int. Cl.: C07D 231/16, C06B 25/34

(54) **Dérivés du 3,4,5-trinitropyrazole, leur préparation et les compositions énergétiques les renfermant**
Derivate von 3,4,5-Trinitropyrazol, ihre Herstellung und die energetischen Zusammensetzungen, die sie enthalten
Derivatives of 3,4,5-trinitropyrazole, their preparation and the energetic compositions containing them

(30) Priorité: 06.06.2008 FR 0853768; 16.12.2008 US 314726
(43) Date de publication de la demande: 09.12.2009
(73) Titulaire: SME, 75015 Paris (FR); Eurenco, 75004 Paris (FR)
(72) Inventeur: Hervé, Grégoire, 60410 Verberie (FR)
(74) Mandataire: Le Roux, Martine

(56) Documents cités:
- WO-A-2008/152525
- LEBEDEV V P ET AL: "Thermochemical and explosive properties of nitropyrazoles" INTERNATIONAL ANNUAL CONFERENCE OF ICT,, vol. 29, 1 janvier 1998 (1998-01-01), pages 180.1-180.13, XP009104460

## Description

La présente invention a pour objet :
- de nouvelles molécules hétérocycliques : de nouveaux dérivés du 3,4,5-trinitropyrazole ;
- la préparation desdites nouvelles molécules;
- les compositions énergétiques renfermant de telles molécules.

Ces nouveaux composés (ces nouvelles molécules) sont particulièrement intéressant(e)s en ce qu'ils (elles) présentent une haute performance énergétique, une faible sensibilité et un bas point de fusion. De surcroit, leur synthèse est tout à fait compatible avec un mode de réalisation industriel.

L'homme du métier recherche en permanence de nouvelles molécules énergétiques présentant un compromis intéressant en termes de performance et de sensibilité tout en conservant un niveau de stabilité thermique répondant aux exigences de la fabrication de chargements pyrotechniques (notamment par la technologie coulé-fondu) et de leur application. De telles molécules présentent avantageusement des caractéristiques de décompositions thermodynamiques permettant leur utilisation dans les domaines de la propulsion et des explosifs.
- Lebedev et al. ont présenté des résultats calculés et/ou mesurés de propriétés physico-chimiques et de performance de composés énergétiques (INTERNATIONAL ANNUAL CONFERENCE OF ICT, vol.29, 1er janvier 1998, p.180.1-108.13). Les formules développées desdites molécules et lesdits résultats figurent dans des tableaux. On note que les synthèses des molécules en cause ne sont pas décrites, ni même suggérées.
- R.D. Schmidt, G.S. Lee, P.F. Pagoria, A.R. Mitchell et R. Gilardi ont décrit, dans J. Heterocyclic Chem, 2001, 38, 122, la synthèse et les propriétés d'un nouvel explosif : le 4-amino-3,5-dinitro-1-pyrazole (LLM-116).
- Les demandes FR 2 917 409 et WO 2008/152525 de la demanderesse, non publiées à la date de priorité de la présente demande, décrivent des composés de formule (I) : 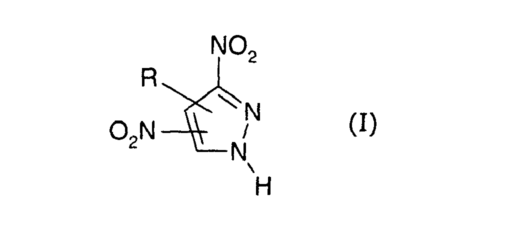
dans laquelle: R = NO₂ NH₂, NF₂, NHOH, OH ou NHNH₂,
différents dudit 4-amino-3,5-dinitro-1-pyrazole,
ainsi que leurs sels.

Ces demandes décrivent plus précisément des composés répondant aux formules (Ia), (I'a), (Ib), (I'b) ci-après : 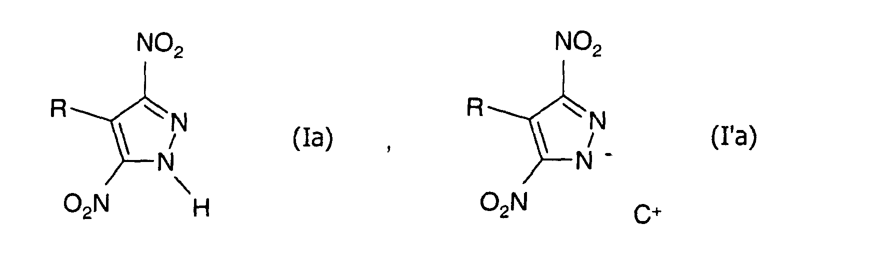 dans laquelle : R = NO₂, NF₂, NHOH, OH ou NHNH₂ ; 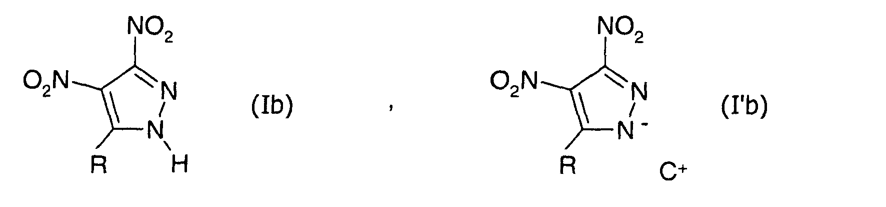 dans laquelle: R = NO₂, NH₂, NF₂, NHOH, OH ou NHNH₂,
ainsi que leur procédé de préparation.

Le schéma réactionnel de la préparation desdits composés de formule (I) est reproduit ci-après.

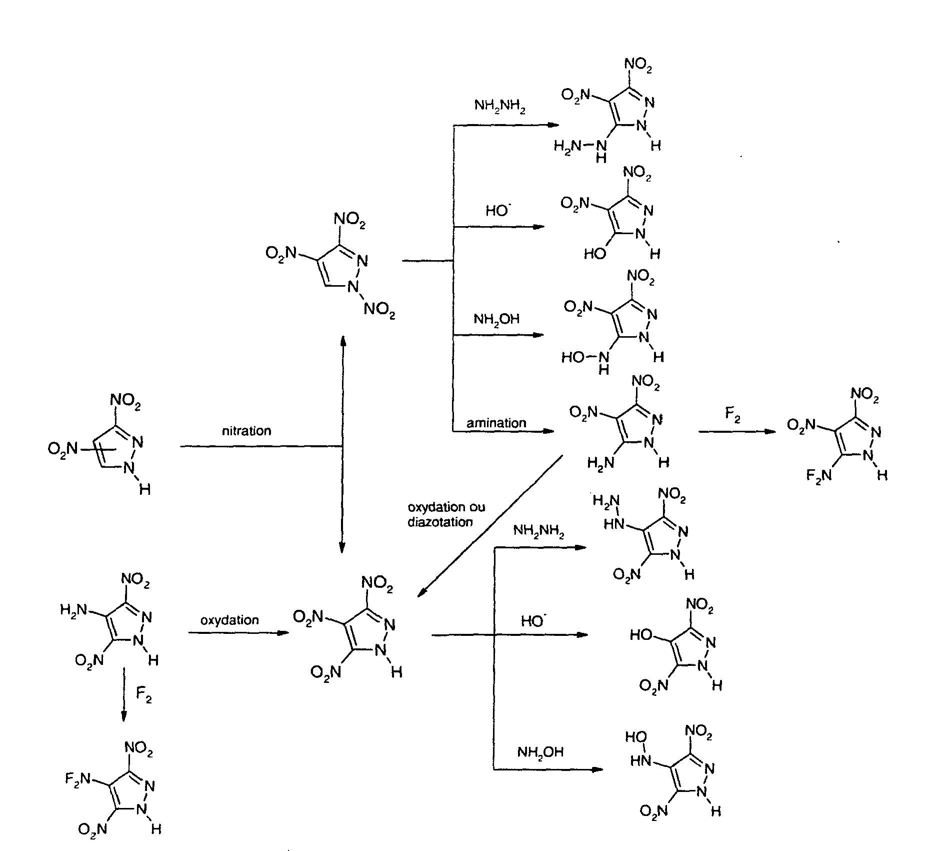

A propos dudit schéma réactionnel, on propose les commentaires ci-après.

Les composés de départ sont des dinitropyrazoles connus. Ils répondent aux formules reproduites ci-dessous et sont décrits dans la littérature. On indique ci-dessous lesdites formules et références bibliographiques correspondantes.
- 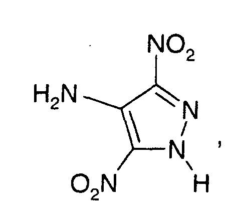 décrit dans J. Heterocyclic Chem. 2001, 38, 122 (voir ci-dessus) ;
- 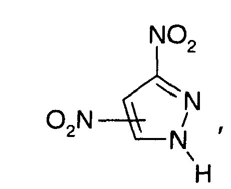 décrits dans J. Org. Chem. 1973, 38, 1777 (par J.W.A.M. Janssen, H.J. Koeners, C.G. Kruse, C.L. Habraken).

Les trinitropyrazoles en cause répondent aux formules ci-après :

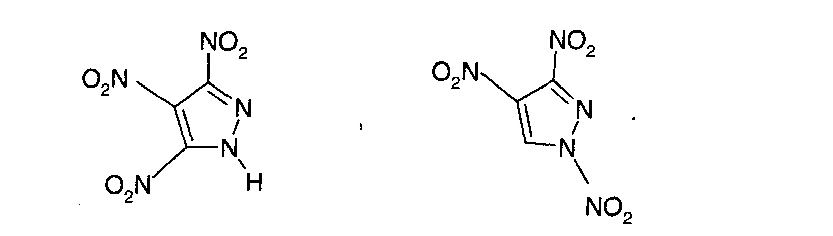

Le premier d'entre eux - le 3,4,5-trinitropyrazole - est un composé de formule (I) qui peut être utilisé comme précurseur pour la préparation d'autres composés de formule (I), ceux de formule (Ia) dans laquelle R=NH-NH₂, OH et NHOH (voir le schéma réactionnel ci-dessus).

Le second d'entre eux - le 1,3,4-trinitropyrazole - est un intermédiaire utile à la préparation de composés de formule (Ib) dans laquelle R=NH-NH₂, OH, NHOH, NH₂ et NF₂. A partir dudit composé de formule (Ib) dans laquelle R=NH₂, on peut accéder audit 3,4,5-trinitropyrazole.

On se propose de décrire, ci-après, chacune des étapes du procédé schématisé ci-dessus.

Le schéma réactionnel montre :
A) pour la préparation du 4-difluoroamino-3,5-dinitropyrazole :
   - la fluoration du 4-amino-3,5-dinitropyrazole ;
B) pour la préparation des autres composés de formule (I) :
   - l'obtention d'un isomère du trinitropyrazole choisi parmi le 3,4,5-trinitropyrazole et le 1,3,4-trinitropyrazole, respectivement, par oxydation du 4-amino-3,5-dinitropyrazole ou nitration du 3,5-dinitropyrazole et par nitration du 3,4-dinitropyrazole ;
   - la mise en oeuvre d'une substitution :
      + sur ledit 1,3,4-trinitropyrazole, pour obtenir les 3,4-dinitropyrazoles substitués en position 5 par le radical R = NH-NH₂, OH, NHOH ou NH₂ ;
      + sur ledit 3,4,5-trinitropyrazole, pour obtenir les 3,5-dinitropyrazoles substitués en position 4 par le radical R = NH-NH₂, OH ou NHOH;
   - la fluoration du 5-amino-3,4-dinitropyrazole obtenu pour obtenir le 5-difluoroamino-3,4-dinitropyrazole.

Ledit schéma réactionnel montre aussi l'obtention du 3,4,5-trinitropyrazole à partir du 5-amino-3,5-dinitropyrazole (par oxydation ou diazotation). Les sels de formule (I'a) et (I'b) sont obtenus par réaction avec une base des composés correspondants de formule (Ia) et (Ib).

Plus précisément :
- Le 4-difluoroamino-3,5-dinitropyrazole est obtenu directement par fluoration du 4-amino-3,5-dinitropyrazole (produit de départ connu). De la même façon, la fluoration du 5-amino-3,4-dinitropyrazole (produit de formule (I) préalablement préparé) conduit au 5-difluoroamino-3,4-dinitropyrazole. Les méthodes classiques et anciennes de fluoration, notamment telles que décrites dans J. Org. Chem. 1968, 33, 1008-11, conviennent parfaitement.
- Le 3,4,5-trinitropyrazole peut être obtenu par trois voies de synthèse différentes :
   + par oxydation du 4-amino-3,5-dinitropyrazole (ou de son isomère le 5-amino-3,4-dinitropyrazole).
      On note en effet que ledit trinitropyrazole peut, de la même façon, être obtenu par oxydation du 5-amino-3,4-dinitropyrazole, préparé préalablement. La réaction d'oxydation, dans l'un ou l'autre contexte, est avantageusement mise en oeuvre dans des peroxydes concentrés. Un mélange concentré : H₂O₂ + H₂SO₄ convient pour l'oxydation d'amines désactivées. Une extraction sélective du milieu est nécessaire. Avantageusement, on utilise le dichlorométhane comme solvant d'extraction. Les exemples 3 et 4 ci-après illustrent une telle oxydation ;
   + par diazotation du 5-amino-3,5-dinitropyrazole (préparé préalablement).
      On opère en solution dans un acide minéral concentré, tel que, par exemple, l'acide sulfurique à 20 %. On peut employer des acides plus concentrés, sans enregistrer d'effet notable sur le rendement. Le sel de diazonium intermédiaire est converti en trinitropyrazole par chauffage de la solution acide en présence de nitrite de sodium. L'exemple 3' ci-après illustre une telle diazotation. La même réaction de diazotation à partir du 4-amino-3,4-dinitropyrazole semble plus délicate à mettre en oeuvre, à cause d'une meilleure stabilité du sel de diazonium (on peut à ce propos se référer à I.L. Dalinger, T.I. Cherkasova, S.A. Shevelev, Mendeleev Commun. 1997, 58) ;
   + par nitration du 3,5-dinitropyrazole, dans des conditions sévères.

Il a en effet été montré que la nitration du 3(5),4-dinitropyrazole, dans des conditions de température modérées (entre 0°C et la température ambiante), conduit à la formation sélective du 1,3,4-trinitropyrazole (voir ci-après) tandis que la nitration du 3,5- dinitropyrazole, dans des conditions sévères (notamment de température), conduit à la formation sélective du 3,4,5-trinitropyrazole. On précise ci-après la préparation du 1,3,4-trinitropyrazole. Pour ce qui concerne la nitration du 3,5-dinitropyrazole, qui conduit directement au 3,4,5-trinitropyrazole, elle est mise en oeuvre en milieu sulfonitrique (H₂SO₄ + HNO₃) ou phosphonitrique (H₃PO₄ + HNO₃), renfermant de 0 à 100 % en masse d'anhydride sulfurique, phosphorique ou nitrique (appelés oléums), à une température comprise entre 50°C et la température d'ébullition du milieu. Cette voie d'accès au 3,4,5-trinitropyrazole - voie d'accès originale, particulièrement avantageuse, à un composé performant - est illustrée à l'exemple 5 ci-après.
- Le 1,3,4-trinitropyrazole est donc obtenu par nitration, dans des conditions de température modérées (voir ci-dessus), du 3(5),4-dinitropyrazole. Une telle nitration peut être mise en oeuvre à la température ambiante dans un mélange acétonitrique (HNO₃ + anhydride acétique), ou équivalent tel que les mélanges HNO₃ + anhydride trifluoroacétique. Une telle nitration en mélange acétonitrique est illustrée à l'exemple A ci-après. Il a par ailleurs été montré, dans le cadre de l'invention, que les solutions de nitronium conviennent également. Les solutions d'ions nitronium peuvent être directement générées à partir des sels de nitronium (comme NO₂BF₄), ou à partir de l'acide nitrique catalysé par H₂SO₄. Une telle mise en oeuvre est illustrée à l'exemple B ci-après.
- A partir du 3,4,5-trinitropyrazole, on peut obtenir, par substitution nucléophile, d'autres composés de formule (I). A partir du 1,3,4-trinitropyrazole, on peut obtenir, par substitution, d'autres composés de formule (I). Les réactions de substitution sont mises en oeuvre en présence de bases inorganiques telles que des solutions d'hydroxydes, d'ammoniac, d'hydrazine et d'hydroxylamine pour former les dinitropyrazoles convenablement substitués de formule (I). Les solutions d'hydroxydes peuvent être générées à partir de sels d'hydrogénocarbonate, de carbonate, d'hydroxyde (soude ou potasse), de nitrite ou même d'eau. Les réactions peuvent être mises en oeuvre dans l'eau ou les solvants organiques tels que la diméthylformamide, le diméthylsulfoxyde, le tétrahydrofurane, l'acétonitrile, le nitrométhane, le sulfolane et leurs mélanges. Lesdites réactions peuvent être mises en oeuvre à une température comprise entre des températures inférieures à 0°C et la température de reflux du solvant utilisé. De préférence, on réalise les réactions de substitution du 3,4,5-trinitropyrazole à des températures élevées (reflux) et les réactions de substitution du 1,3,4-trinitropyrazole à basse température. En fait, comme décrit dans l'article de Habraken et Poels (J. Org. Chem. 1977, 42, 2893), les réactions de substitution sur les dérivés du 1,4-dinitropyrazole, tel le 1,3,4-trinitropyrazole, conduisent à la fonctionnalisation de la position 5 du pyrazole et à une substitution du N-nitro. En revanche, dans des conditions plus poussées, la substitution nucléophile sur le 3,4,5-trinitropyrazole intervient de façon inattendue sur le carbone (4) du pyrazole.

Pour l'obtention du 5-amino-3,4-dinitropyrazole (qui peut donc être utilisé comme précurseur du 3,4,5-trinitropyrazole), la réaction de substitution concernée est une amination. Les techniques d'amination connues sont nombreuses. On peut citer par exemple la réaction avec l'ammoniac (mentionnée ci-dessus), la méthode de Gabriel (passage par le dérivé phtalamide) et la réduction d'un dérivé azoture. De manière avantageuse, on peut ainsi préparer, de façon sélective, le 5-amino-3,4-dinitropyrazole, en deux étapes :
- la réaction de sels d'azoture sur le 1,3,4-trinitropyrazole pour former le 5-azido-3,4-dinitropyrazole, (voir, pour illustration, l'exemple C ci-après) ;
- la réduction dudit 5-azido-3,4-dinitropyrazole par des méthodes classiques, telles que l'action d'une phosphine trisubstituée ou de l'acide thiolacétique (voir, pour illustration, les exemples 1 et 2 ci-après).

Ces deux étapes sont schématisées ci-après :

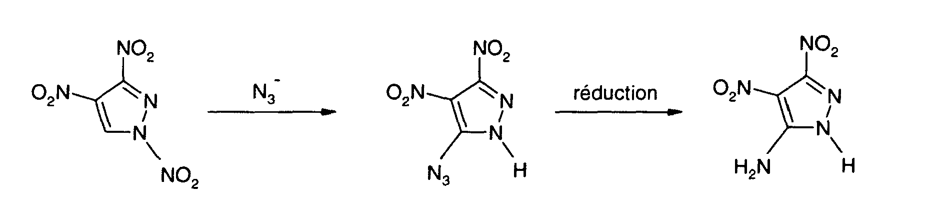

On rappelle que ledit 5-amino-3,4-dinitropyrazole :
est susceptible, par oxydation ou diazotation, de donner le 3,4,5-trinitropyrazole;
est susceptible aussi, par fluoration, de donner le 4-difluoroamino-3,5-dinitropyrazole (voir ci-dessus).

Ce procédé de préparation des composés de formule (I) a été repris en détail ci-dessus dans la mesure où il englobe la préparation du 3,4,5-trinitropyrazole, précurseur utile à la préparation des composés de la présente invention.

La présente invention concerne, selon son premier objet, une nouvelle classe de molécules énergétiques. Ces molécules énergétiques sont des dérivés nouveaux du 3,4,5-trinitropyrazole. Lesdits dérivés répondent à la formule (Ic) ci-après : 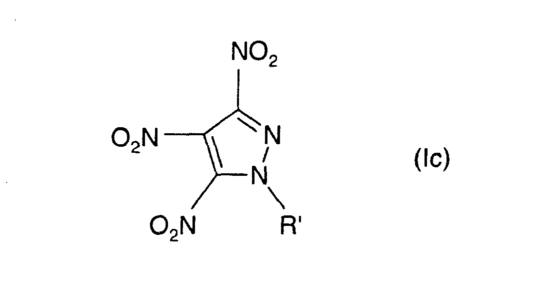 dans laquelle R'=NH₂ ou un groupe alkyle en C₁-C₄, linéaire ou ramifié, éventuellement substitué par au moins un groupe hydroxy et/ou un fluor.

R' est un groupe amino ou un groupe alkyle inférieur (comportant de 1 à 4 atomes de carbone), éventuellement substitué. Ledit groupe alkyle inférieur peut être linéaire ou ramifié. Il est choisi parmi les groupes méthyle, éthyle, n-propyle, iso-propyle, n-butyle, iso-butyle et tertio-butyle, non substitué ou substitué par au moins un groupe hydroxy et/ou au moins un fluor. Il peut notamment consister en un groupe alkyle inférieur perfluoré. Selon une variante avantageuse, R' est un groupe amino (-NH₂) ou un groupe méthyle (-CH₃). Il consiste très avantageusement en un groupe méthyle.

Les composés de l'invention, de formule (Ic), présentent un point de fusion plus bas que celui du 3,4,5-trinitropyrazole, plus bas généralement que ceux des composés décrits dans les demandes FR 2 917 409 et WO 2008/152525 (composés non substitués sur l'azote en position 1). Leur pouvoir énergétique, plus ou moins diminué, demeure intéressant de par la présence des trois groupes nitro. Lesdits composés peuvent, en tout état de cause, être dopés par leur utilisation conjointe avec au moins une autre molécule énergétique plus performante, par exemple de type RDX (cyclotriméthylène trinitramine ou hexogène), HMX (cyclotétra-méthylène tétranitramine ou octogène), CL20 (2,4,6,8,10,12-hexanitro-hexaazaisowurtzitane ou HNIW), autre hétérocycle azoté connu à ce jour (voir plus loin).

Le bas point de fusion desdits composés de l'invention est particulièrement intéressant. Il autorise la formulation de chacun desdits composés, en tant qu'explosif coulé-fondu, par un procédé (de fabrication d'explosifs) familier à l'homme du métier. Ledit procédé ne fait pas intervenir de liant, ne comprend pas de compression de poudre. Il consiste principalement à faire fondre le(s) composé(s) énergétique(s) en cause par simple élévation de la température puis à laisser cristalliser la masse fondue par simple refroidissement. Avantageusement, la masse fondue est chargée en au moins une autre molécule énergétique plus performante (notamment, des types identifiés ci-dessus).

Le N-méthyl-3,4,5-trinitropyrazole est un composé de l'invention très intéressant en ce qu'il associe un point de fusion particulièrement bas (91,3°C) à des propriétés énergétiques intéressantes (il présente notamment une balance en oxygène nettement supérieure à celle du TNT).

Dans le cadre de son premier objet, la présente invention englobe les composés de formule (I) identifiés ci-après :
- le N-méthyl-3,4,5-trinitropyrazole, et
- le N-amino-3,4,5-trinitropyrazole.

Ces composés de formule (I) sont particulièrement préférés.

Les composés de l'invention sont des composés énergétiques peu sensibles, performants. Leur niveau de performance énergétique équivaut, voire dépasse pour certains, celui de l'octogène, pour des niveaux de sensibilité comparables à ceux de l'ONTA. Lesdits composés de l'invention sont donc particulièrement intéressants :
en ce qu'ils sont à la fois énergétiques et peu sensibles ;
en ce qu'ils répondent, de façon particulièrement avantageuse, au cahier des charges ci-après :
   - performances énergétiques supérieures à celles de l'ONTA,
   - faible sensibilité,
   - stabilité thermique adéquate pour les applications envisagées,
   et
   - bas point de fusion.

Selon son deuxième objet, la présente invention concerne la préparation des nouveaux composés de formule (Ic) de l'invention.

Pour la préparation d'un 3,4,5-trinitropyrazole N-substitué selon l'invention, on utilise donc le 3,4,5-trinitropyrazole. On prépare l'anion dudit 3,4,5-trinitropyrazole, en présence d'une base et on met en oeuvre une substitution nucléophile sur celui-ci.

Le schéma réactionnel, pour la préparation des composés N-substitués de l'invention, est donc le suivant : 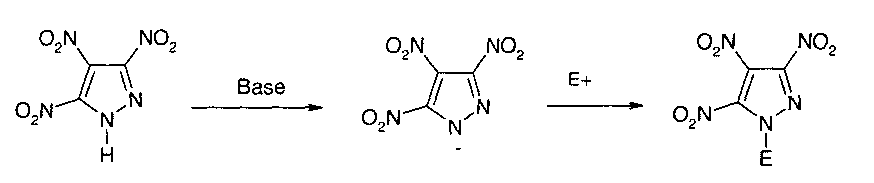
1) Le 3,4,5-trinitropyrazole (produit de départ, précurseur des composés N-substitués de l'invention) peut donc être obtenu par l'une quelconque des voies (oxydation du 4-amino-3,5-dinitropyrazole ou du 5-amino-3,4-dinitropyrazole, nitration du 3,5-dinitropyrazole ou diazotation du 5-amino-3,5-dinitropyrazole) englobées dans le schéma réactionnel de préparation des composés de formule (I) montré ci-dessus. Lesdites voies sont reproduites ci-après. 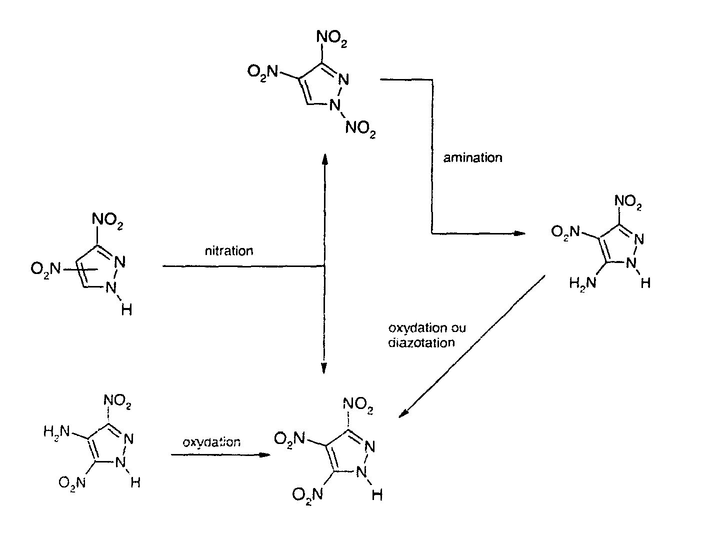 Pour des précisions à propos de chacune desdites voies, on renvoie aux commentaires ci-dessus, en référence au schéma global de préparation des composés de formule (I).
2) En présence d'une base minérale ou organique, telle NaOH, KOH, NaH, un alcoolate, la pyridine, on obtient l'anion du 3,4,5-trinitropyrazole.
3) Celui-ci, en présence d'un électrophile convenable, subit une substitution nucléophile qui génère le composé attendu.

Le composé méthyle peut ainsi être obtenu par action d'un agent de méthylation conventionnel, tel l'iodure de méthyle, le triflate de méthyle, le diazométhane, les sels de triméthyl oxonium. Les autres composés alkyles peuvent être obtenus de manière analogue.

Le composé amino peut ainsi être obtenu par amination électrophile, par action d'un agent d'amination électrophile conventionnel, tel la chloramine (NH₂Cl), l'acide hydroxylamine-O-sulfonique (HOSA, NH₂-O-SO₃H), le dinitrophénoxy-O-hydroxylamine, le trinitrophénoxy-O-hydroxylamine (ou Pic-O-NH₂=hydroxylamine de l'acide picrique).

Les conditions de mise en oeuvre de la substitution nucléophile sont *per se* classiques. Ce qui est original, c'est la nature exacte de l'anion en cause.

Les nouveaux composés de formule (Ic) de l'invention :
- tels qu'identifiés ci-dessus, de manière générale et de manière spécifique, et/ou
- tels que susceptibles d'être obtenus par le procédé décrit ci-dessus
   présentent des caractéristiques énergétiques intéressantes (en référence au compromis : sensibilité/performance et à la stabilité thermique). Leurs performances sont intéressantes aussi bien pour des applications propulsives que des applications explosives.

Les composés de l'invention constituent clairement des concurrents intéressants aux molécules énergétiques insensibles que sont l'ONTA, le TATB et le I-*RDX^{®}. Ils sont des candidats sérieux pour des applications de type MURAT en explosifs. Par ailleurs, ils sont particulièrement adaptés à l'obtention de chargements explosifs peu sensibles par la technologie coulé-fondu.

Selon son troisième objet, la présente invention concerne donc des compositions énergétiques renfermant au moins un composé de formule (Ic) tel que décrit ci-dessus.

La détermination de la quantité efficace dudit au moins un composé est à la portée de l'homme du métier, au vu du débouché exact souhaité pour lesdites compositions énergétiques. Lesdites compositions énergétiques peuvent notamment consister en des compositions explosives ou des compositions propulsives. Ces deux types de compositions énergétiques (avec des molécules énergétiques conventionnelles) sont familiers à l'homme du métier.

Les compositions énergétiques de l'invention du premier type - compositions explosives - renferment ou ne renferment pas de liant (inerte ou énergétique). Elles renferment généralement au moins 20 % en masse d'au moins un composé de formule (Ic), très généralement au moins 50 % en masse d'un tel composé de formule (I). Elles sont tout à fait susceptibles de renfermer entre 90 % en masse et 100 % en masse, d'au moins un tel composé de formule (Ic).

Les compositions énergétiques de l'invention du second type - compositions propulsives - renferment un liant, inerte ou énergétique. Elles renferment généralement au plus 80 % en masse d'au moins un composé de formule (Ic), très généralement au plus 50 % en masse d'un composé de formule (Ic).

Les compositions énergétiques de l'invention, pour lesquelles on a précisé ci-dessus, de façon nullement limitative, la teneur en composé(s) de l'invention (composé(s) de formule (Ic)) peuvent être obtenues par compression d'une poudre, par malaxage, coulée puis réticulation en présence d'un liant polymérique ou par coulé-fondu.

Les compositions énergétiques renfermant au moins un composé de l'invention (qui présente un bas point de fusion) sont avantageusement de type coulé-fondu. Le N-méthyl-3,4,5-trinitropyrazole est très avantageusement présent dans ce type de compositions (type coulé-fondu).

Lesdites compositions énergétiques de type coulé-fondu renferment au moins un tel composé (généralement d'un tel composé) ou au moins un tel composé (généralement un tel composé) avec au moins une autre molécule énergétique connue plus performante. Ladite au moins une autre molécule énergétique est avantageusement choisie parmi les molécules énergétiques conventionnelles telles le HMX, le RDX, le CL20, un autre hétérocycle azoté (voir ci-dessus).

La présence d'une telle au moins une autre molécule énergétique (plus performante) n'est pas obligatoire, elle est avantageuse. Généralement, lorsqu'elle est présente, une telle au moins une autre molécule énergétique l'est de 10 à 80 % en masse. Sa présence à un taux de 60% en masse est tout particulièrement préconisée.

On se propose maintenant d'illustrer l'invention par les exemples ci-après.

Les exemples **6** et **7** illustrent la préparation de composés N-substitués de l'invention à partir du 3,4,5-trinitropyrazole. Ledit trinitropyrazole est obtenu :
- à partir du 5-amino-3,4-dinitropyrazole par oxydation (exemple 3) ou par diazotation (exemple 3') ; ledit 5-amino-3,4-dinitropyrazole étant obtenu par réduction du 5-azido-3,4-dinitropyrazole (exemples 1 et 2), lui-même obtenu à partir du 1,3,4-trinitropyrazole (exemple C), lui-même obtenu par nitration du 3,4-dinitropyrazole (exemples A et B) ; ou
- à partir du 4-amino-3,5-dinitropyrazole par oxydation (exemple 4) ; ou encore
- à partir du 3,5-dinitropyrazole par nitration (exemple 5).

### Exempte A

On introduit 200 mg de 3,4-dinitropyrazole dans 7 mL d'anhydride acétique contenant 1,4 mL (2,13 g) d'acide nitrique concentré à 0°C. On laisse remonter la température à 15°C, puis on laisse agiter à cette température pendant 18 heures. On verse le milieu réactionnel sur 70 g de glace, puis on extrait le produit au dichlorométhane. La phase organique chlorée est séchée sur sulfate de magnésium puis filtrée et concentrée sous vide. 200 mg de produit brut sont ainsi récupérés. Le 1,3,4-trinitropyrazole est purifié sur gel de silice par élution au mélange hexane/AcOEt (2/0,5, v/v). 34 mg de liquide incolore pur sont récupérés (13% de rendement), RMN ¹H (acétone) : 8,49 (s, CH); RMN ¹³C (acétone) : 128,6 (CH), 128,4 (CNO₂, large), 145,1 (t, CNO₂); RMN ¹⁴N (acétone) : 310,3, 314, 351,5 ppm. DSC : température de décomposition : 191°C.

### Exemple B

On introduit, sous balayage d'argon à 0°C, 200 mg de 3,4-dinitropyrazole dans 20 mL d'acétonitrile anhydre contenant 193 mg de tétrafluoroborate de nitronium. On laisse ensuite réagir à température ambiante pendant 18 heures. Le milieu réactionnel est ensuite concentré sous vide, puis traité avec 30 mL d'eau glacée et extrait au dichlorométhane. La phase de récupération et de purification est identique à celle décrite dans l'exemple A. Après purification, 121 mg de liquide pur incolore sont ainsi récupérés (rendement 47%).

### Exemple C

On coule lentement une solution de 1,3,4-trinitropyrazole, décrit dans les exemples 1 et 2, dilué dans 5 mL de tétrahydrofurane sur une suspension agitée d'azoture de sodium contenu dans du diméthylsulfoxyde (28 mL) à température ambiante. Après 1 heure d'agitation, le milieu réactionnel est versé sur 280 g de glace, puis acidifié jusqu'à pH égal à 1 à l'aide d'une solution d'acide chlorhydrique à 37%. On extrait le milieu avec 60 mL d'éther diéthylique. La phase organique est récupérée, puis lavée avec HCl 0,1 N, séchée sur sulfate de magnésium, et concentrée sous vide. Le produit est ensuite trituré dans 10 mL de dichlorométhane jusqu'à obtention d'un solide jaune. Le 5-azido-3,4-dinitropyrazole est filtré puis séché sous vide pendant 30 minutes. On récupère ainsi 220 mg de solide jaune utilisé tel quel dans la phase suivante (rendement 23%). RMN ¹H (acétone) : pas de signal. RMN ¹³C (acétone) (ppm) : 118,1 (Q), 140,6 (Q), 150,1 (Q). RMN ¹⁴N (acétone) (ppm) : 357,1 (NO₂), 355,4 (NO₂), 310,3 (N₃), 231. DSC : température de fusion 141,3°C ; température de décomposition 156,7°C.

### Exemple 1

200 mg de 5-azido-3,4-dinitropyrazole sont réduits dans 10 mL d'acide thiolacétique à température ambiante pendant 2 jours. On verse ensuite 20 mL d'hexane pour faire précipiter le produit. 50 mg de solide jaune sont ainsi récupérés par filtration (rendement 28%).

La formule développée dudit produit ainsi que les résultats d'analyses physico-chimiques (RMN ¹H, ¹³C, ¹⁴N et SM) confirmant la structure figurent dans le tableau 1 ci-après.

### Exemple 2

On ajoute, en une fois et à température ambiante, 287 mg de triphénylphosphine dans 20 mL de tétrahydrofurane contenant 200 mg de 5-azido-3,4-dinitropyrazole. On laisse agiter 18 heures à température ambiante, puis on ajoute 5 mL d'eau distillée. On laisse réagir 20 heures à reflux. On ajoute ensuite de la soude à 10% pour augmenter le pH jusqu'à 10-11. Le milieu réactionnel est lavé à l'acétate d'éthyle 2 fois (20 mL), puis acidifié jusqu'à un pH acide de 1 à l'aide d'acide chlorhydrique à 10%. Le produit est ensuite extrait avec 20 mL d'éther diéthylique (3 fois). Les phases éthérées sont rassemblées, puis lavées avec une solution saturée de chlorure de sodium, séchées sur sulfate de sodium, et concentrées sous vide. Le produit est ensuite trituré dans 20 mL de dichlorométhane pour être filtré et séché. 65 mg de solide rouge sont ainsi récupérés (rendement 51%).

La formule développée dudit produit ainsi que les résultats d'analyses physico-chimiques (RMN ¹H, ¹³C, ¹⁴N et SM) confirmant la structure figurent dans le tableau 1 ci-après.

### Exemple 3

600 mg de 5-amino-3,4-dinitropyrazole sont ajoutés en une fois dans un mélange bien agité de persulfate de sodium (3 g), d'eau oxygénée à 60% (2,5 g) et d'acide sulfurique concentré (4 g) à température ambiante. Le milieu réactionnel est agité pendant 8 heures puis extrait 5 fois au dichlorométhane (5*20 mL). Les phases chlorées sont rassemblées, séchées sur sulfate de magnésium, filtrées puis concentrées sous vide. 217 mg de produit pur sont ainsi récupérés (rendement 31%).

La formule développée dudit produit ainsi que les résultats d'analyses physico-chimiques (RMN ¹H, ¹³C, ¹⁴N et SM) confirmant la structure figurent dans le tableau 1 ci-après.

### Exemple 3'

On ajoute, rapidement, à 0-10°C, 650 mg de nitrite de sodium dans une solution d'acide sulfurique à 20% (5 mL) contenant 145 mg de 5-amino-3,4-dinitropyrazole. On laisse réagir à cette température pendant 1 heure, puis le milieu froid est versé sur 40 mL de nitrite de sodium aqueux à 10% maintenu à 20°C. Le mélange est ensuite chauffé à 50-60°C pendant 30 minutes jusqu'à disparition complète de l'effervescence. Le nitrite de sodium en excès est ensuite neutralisé par addition d'acide sulfurique concentré. Le milieu réactionnel est extrait 3 fois avec de l'acétate d'éthyle. Les phases organiques sont rassemblées, lavées avec une saumure, séchées sur sulfate de magnésium, filtrées, puis concentrées. Le 3,4,5-trinitropyrazole est obtenu sous la forme d'une gomme orangée avec un rendement brut de 80%. Les analyses RMN, IR sont conformes à celles obtenues avec le produit de l'exemple 3.

### Exempte 4

600 mg de 4-amino-3,5-dinitropyrazole sont ajoutés en une fois dans un mélange bien agité de persulfate de sodium (3 g), d'eau oxygénée à 60% (2,5 g) et d'acide sulfurique concentré (4 g) à température ambiante. Le milieu réactionnel est agité pendant 8 heures puis extrait 5 fois au dichlorométhane (5*20 mL). Les phases chlorées sont rassemblées, séchées sur sulfate de magnésium, filtrées puis concentrées sous vide. 325 mg de produit pur sont ainsi récupérés (rendement 47%).

La formule développée dudit produit ainsi que les résultats d'analyses physico-chimiques (RMN ¹H, ¹³C, ¹⁴N et SM) confirmant la structure figurent dans le tableau 1 ci-après.

### Exemple 5

202 mg de 3,5-dinitropyrazole sont dissous dans 6 g d'acide sulfurique concentré. 4 g d'acide nitrique fumant sont ajoutés rapidement à température ambiante. On ajoute ensuite à l'aide d'une ampoule de coulée 6 g d'oléum sulfurique à 60% en SO₃ (titre massique). On laisse monter la température du milieu réactionnel. Le milieu est ensuite chauffé pendant 1 heure à 70°C. On laisse refroidir à température ambiante avant d'hydrolyser le milieu réactionnel dans 30 g de glace. Le mélange est extrait 3 fois avec de l'acétate d'éthyle. Les phases organiques sont rassemblées, lavées avec une solution saturée de chlorure de sodium, séchées sur sulfate de magnésium, filtrées, puis concentrées sous vide. 245 mg de produit pur sont ainsi récupérés (rendement 95%).

La formule développée dudit produit ainsi que les résultats d'analyses physico-chimiques (RMN ¹H, ¹³C, ¹⁴N et SM) confirmant la structure figurent dans le tableau 1 ci-après.
• On propose ci-après ledit tableau 1.

**Tableau 1**

| exemple | 1, 2 | 3,3',4,5 |
|---|---|---|
| formule | 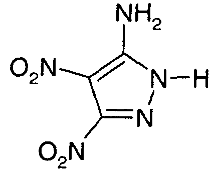 | 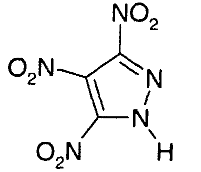 |
| RMN ¹H (ppm) | 7,31 (large) | 12,3 (large) |
| RMN ¹³C (ppm) | 109,8, 148,7, 150,3 | 122,6 (t), 143,2 |
| RMN ¹⁴ N (ppm) | 359 | 348,9 |
| SM | IC⁺ (NH₃) : 174 | IE : 203 IC (NH₃) : 202 |
| Température de fusion par DSC (°C) | 204 | 188 |

On a par ailleurs confirmé la structure des produits obtenus aux exemples :
1 et 2 : le 5-amino-3,4-dinitropyrazole
3, 4 et 5 : le 3,4,5-trinitropyrazole
par diffraction aux rayons X.

### Exemple 6

On introduit à 10°C sous balayage d'argon 17 mg d'hydrure de sodium à une solution d'acétonitrile anhydre (10 mL) contenant 130 mg de 3,4,5-trinitropyrazole. Le dégagement d'hydrogène est immédiat et important. On laisse agiter le milieu réactionnel en laissant revenir à température ambiante. 400 µL d'iodure de méthyle sont alors additionnés rapidement, puis le mélange est chauffé à reflux pendant 2 heures. Le milieu brunit au cours du temps par formation d'iode. On laisse ensuite refroidir avant de noyer le milieu dans 50 mL d'eau distillée. Le produit est extrait par trois fois au chloroforme. Les phases organiques sont rassemblées, séchées sur sulfate de magnésium, évaporées pour produire 105 mg de liquide jaune. Le produit (N-méthyl-3,4,5-trinitropyrazole) est purifié sur gel de silice. 91 mg (66% de rendement) de solide jaune très pâle sont ainsi collectés. RMN ¹H (CDCl₃) (ppm) : 4,42 (s). RMN ¹³C (CDCl₃) (ppm) : 43,2 (CH₃), 123,5 (t), 137,7 (t), 148,8 (m). RMN ¹⁴N (CDCl₃) (ppm) : 343,9, 345,3, 348,0. DSC : température de fusion : 91,3°C ; température de vaporisation : 243°C.

### Exemple 7

24 mg d'hydrure de sodium (0,98 mmol) sont ajoutés à une solution froide (0°C) de 3,4,5-trinitropyrazole dilué dans 10 mL d'acétonitrile anhydre. Après quelques minutes, on ajoute le Pic-*O*-NH₂ puis on laisse revenir à température ambiante. Le milieu est agité pendant 3 jours. Le picrate de sodium (solide jaune) précipitant dans le milieu est filtré. Le filtrat est ensuite évaporé à sec. Le produit est ensuite purifié sur gel de silice avec un mélange heptane/AcOEt (3/1, v/v). Les fractions contenant le produit sont rassemblées puis concentrées sous vide à 20°C jusqu'à obtenir la précipitation du produit. 55 mg de solide blanc (N-amino-3,4,5-trinitropyrazole) sont ainsi collectés. DSC : température de fusion : 130,5°C ; température de décomposition : 239°C. RMN ¹H (CD₃NO₂) (ppm) : 7,07 (s (large)). RMN ¹³C (CD₃NO₂) (ppm) : 123,1 (t, *J*_{CN}= 17 Hz, C(4)-NO₂), 136,5 (t, *J*_{CN}= 17 Hz, C-NO₂), 140,8 (large, C-NO₂). RMN INEPT ¹⁵N (CD₃NO₂) (ppm) : -288,7 (NH₂, *J*_{NH}= 74 Hz). RMN ¹⁴N (CD₃NO₂)(ppm) : -31,7, -33,3 (fin), -36,7 (fin). RMN ¹H (CD₃CN) (ppm) : 6,92 (s (large)). RMN ¹⁴N (CD₃CN) (ppm) : -28,3, -29,8 (fin), -33,4 (fin), -68,2. SM (IE): 218.

On indique dans le tableau 2 ci-après les performances :
- de composés de l'art antérieur (HMX, RDX, ONTA, CL20),
- des composés des exemples 1 (et 2), 3 (3', 4 et 5) ci-dessus, et
- de composés de l'invention (composés des exemples **6** et **7** ci-dessus).

Les performances ont été calculées en explosifs, puis en propulsion, pour des applications en propergol, en présence de liants plastifiés aux huiles nitrées. Les polymères constitutifs desdits liants sont de type énergétique (polyazoture de glycidyle : PAG) ou inerte de type polyéther (polyéther hydroxytéléchélique : HTPE).

Les résultats de performance en explosifs et en propulsion (calculés pour des compositions discrètes sans ajout de perchlorate d'ammonium et d'aluminium) sont donc rassemblés dans le tableau 2 ci-après.

**Tableau 2**

| Composés | Enthalpie de formation (kcal/mol) | Densité | Vitesse de détonation (m/s) E/E_{HMX} (V/Vo= 2)* | Impulsion spécifique Composition discrète (15% HTPE) (s) | Impulsion spécifique Composition discrète (30% PAG) (s) |
|---|---|---|---|---|---|
| HMX | 20,1 | 1,908 | 9321 | 249,9 | 253,8 |
| | | | 100% | | |
| RDX | 16,7 | 1,823 | 9908 | 250,6 | 254,3 |
| | | | 92% | | |
| ONTA | -25,7 | 1,910 | 8544 | / | / |
| | | | 72% | | |
| CL20 | / | / | / | 260 | 261 |
| Exs. 1 et 2 | 14,4 | 1,872 | 8640 | 220,4 | 230,1 |
| | | | 82% | | |
| Exs. 3, 3', 4 et 5 | 34,1 | 1,867 | 9253 | 261,3 | 261,9 |
| | | | 96% | | |
| Ex. **6** | 18 | 1,87 | 8845 | 239 | 245 |
| | | | 97% | | |
| Ex. **7** | 41 | 1,90 | 9682 | 258 | 264,9 |
| | | | 109% | | |

Les résultats des calculs du tableau 2 démontrent l'intérêt des dérivés N-amino et N-méthyl du 3,4,5-trinitropyrazole à la fois comme charge énergétique de compositions propulsives et de compositions explosives.

Les impulsions spécifiques calculées avec le N-amino-3,4,5-trinitropyrazole sont en effet du même ordre de grandeur que, voire supérieures à, celles calculées pour la molécule en développement la plus performante (CL20).

Le faible point de fusion du N-méthyl-3,4,5-trinitropyrazole permet d'envisager son application par le procédé simple de mise en oeuvre coulé-fondu (voir ci-dessus). Les compositions actuelles emploient comme charge fusible le TNT qui offre des performances modestes alors que son remplacement par le N-méthyl-3,4,5-trinitropyrazole permettrait assurément d'accéder aux performances des explosifs composites les plus puissants.

## Revendications

1. Dérivé du 3,4,5-trinitropyrazole de formule (Ic) : 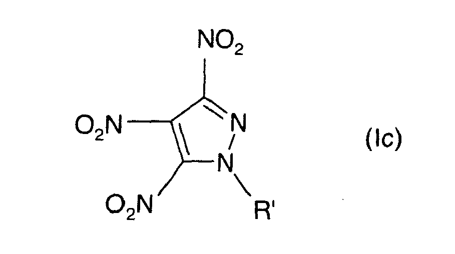 dans laquelle R'=NH₂ ou un groupe alkyle en C₁-C₄, linéaire ou ramifié, éventuellement substitué par au moins un groupe hydroxy et/ou un fluor.

2. Dérivé du 3,4,5-trinitropyrazole selon la revendication 1, choisi parmi :
- le N-méthyl-3,4,5-trinitropyrazole, et
- le N-amino-3,4,5-trinitropyrazole.

3. Procédé pour la préparation d'un dérivé du 3,4,5-trinitropyrazole selon la revendication 1 ou 2, **caractérisé en ce qu'**il comprend :
- l'obtention du 3,4,5-trinitropyrazole ;
- l'obtention de son anion, en présence d'une base ;
- la mise en oeuvre d'une substitution nucléophile sur ledit anion.

4. Procédé selon la revendication 3, **caractérisé en ce qu'**il comprend l'obtention dudit 3,4,5-trinitropyrazole par oxydation du 4-amino-3,5-dinitropyrazole ou du 5-amino-3,4-dinitropyrazole, nitration du 3,5-dinitropyrazole ou diazotation dudit 5-amino-3,4-dinitropyrrazole.

5. Procédé selon la revendication 4, **caractérisé en ce que** ledit 5-amino-3,4-dinitropyrrazole est obtenu par amination du 1,3,4-trinitropyrazole, lui même obtenu par nitration du 3,4-dinitropyrazole.

6. Composition énergétique **caractérisée en ce qu'**elle renferme au moins un composé de formule (I) selon la revendication 1 ou 2.

7. Composition énergétique selon la revendication 6, **caractérisée en ce qu'**elle renferme au moins 20 % en masse d'au moins un composé de formule (I) selon la revendication 1 ou 2.

8. Composition énergétique selon la revendication 6 ou 7, **caractérisée en ce qu'**elle renferme de 90 % à 100 % en masse d'au moins un composé de formule (I) selon la revendication 1 ou 2.

9. Composition énergétique selon la revendication 6, **caractérisée en ce qu'**elle renferme au plus 80 % en masse d'au moins un composé de formule (I) selon la revendication 1 ou 2, dans un liant.

10. Composition énergétique selon l'une quelconque des revendications 6 à 9, **caractérisée en ce qu'**elle consiste en un explosif coulé-fondu renfermant au moins un composé de formule (I) selon la revendication 1 ou 2.

11. Composition énergétique selon la revendication 10, **caractérisée en ce qu'**elle renferme au moins une autre molécule énergétique

12. Composition énergétique selon la revendication 11, **caractérisée en ce qu'**elle renferme au moins une autre molécule énergétique à une teneur comprise entre 10 et 80 % en masse.

## Claims

1. A 3,4,5-trinitropyrazole derivative of formula (Ic): 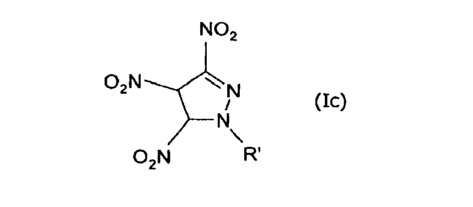 in which R' = NH₂ or a linear or branched C₁-C₄ alkyl group which is optionally substituted by at least one hydroxy group and/or one fluorine.

2. The 3,4,5-trinitropyrazole derivative according to claim 1, selected from:
- N-methyl-3,4,5-trinitropyrazole; and
- N-amino-3,4,5-trinitropyrazole.

3. A process for preparing a 3,4,5-trinitropyrazole derivative according to claim 1 or claim 2, **characterized in that** it comprises:
- obtaining the 3,4,5-trinitropyrazole;
- obtaining its anion, in the presence of a base;
- carrying out a nucleophilic substitution on said anion.

4. The process according to claim 3, **characterized in that** it comprises obtaining said 3,4,5-trinitropyrazole by oxidizing 4-amino-3,5-dinitropyrazole or 5-amino-3,4-dinitropyrazole, by nitrating 3,5-dinitropyrazole or by diazotizing said 5-amino-3,4-dinitropyrazole.

5. The process according to claim 4, **characterized in that** said 5-amino-3,4-dinitropyrazole is obtained from amination of 1,3,4-trinitropyrazole, itself obtained from nitration of 3,4-dinitropyrazole.

6. An energetic composition **characterized in that** it contains at least one compound of formula (I) according to claim 1 or claim 2.

7. The energetic composition according to claim 6, **characterized in that** it contains at least 20% by weight of at least one compound of formula (I) according to claim 1 or claim 2.

8. The energetic composition according to claim 6 or claim 7, **characterized in that** it contains from 90% to 100% by weight of at least one compound of formula (I) according to claim 1 or claim 2.

9. The energetic composition according to claim 6, **characterized in that** it contains not more than 80% by weight of at least one compound of formula (I) according to claim 1 or claim 2, in a binder.

10. The energetic composition according to any one of claims 6 to 9, **characterized in that** it consists in a melt-cast explosive containing at least one compound of formula (I) according to claim 1 or claim 2.

11. The energetic composition according to claim 10, **characterized in that** it contains at least one other energetic molecule.

12. The energetic composition according to claim 11, **characterized in that** it contains at least one other energetic molecule, at a level of between 10% and 80% by weight.

## Patentansprüche

1. Derivat des 3,4,5-Trinitropyrazol der Formel (Ic): 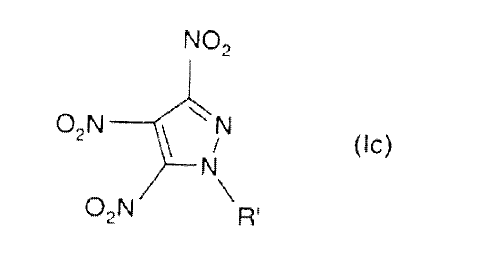 worin R`=NH₂ oder eine C₁-C₄-Alkylgruppe, linear oder verzweigt, eventuell substituiert durch wenigstens eine Hydroxygruppe und/oder ein Fluor, ist.

2. Derivat des 3,4,5-Trinitropyrazol nach Anspruch 1, das ausgewählt ist aus:
- N-Methyl-3,4,5-trinitropyrazol und
- N-Amino-3,4,5-trinitropyrazol.

3. Verfahren zur Herstellung eines Derivats des 3,4,5-Trinitropyrazol nach Anspruch 1 oder 2, **dadurch gekennzeichnet, daß** es umfaßt:
- den Erhalt des 3,4,5-Trinitropyrazol,
- den Erhalt seines Anions, in Gegenwart einer Base,
- die Durchführung einer nukleophilen Substitution an dem Anion.

4. Verfahren nach Anspruch 3, **dadurch gekennzeichnet, daß** es den Erhalt des 3,4,5-Trinitropyrazol durch Oxidation des 4-Amino-3,5-dinitropyrazol oder des 5-Amino-3,4-dinitropyrazol, Nitrierung des 3,5-Dinitropyrazol oder Diazotierung des 5-Amino-3,4-dinitropyrazol umfaßt.

5. Verfahren nach Anspruch 4, **dadurch gekennzeichnet, daß** das 5-Amino-3,4-dinitropyrazol durch Aminierung des 1,3,4-Trinitropyrazol erhalten wird, das selbst durch Nitrierung des 3,4-Dinitropyrazol erhalten wird.

6. Energetische Zusammensetzung, **dadurch gekennzeichnet, daß** sie wenigstens eine Verbindung der Formel (I) nach Anspruch 1 oder 2 enthält.

7. Energetische Zusammensetzung nach Anspruch 6, **dadurch gekennzeichnet, daß** sie wenigstens 20 Masse-% wenigstens einer Verbindung der Formel (I) nach Anspruch 1 oder 2 enthält.

8. Energetische Zusammensetzung nach Anspruch 6 oder 7, **dadurch gekennzeichnet, daß** sie 90 bis 100 Masse-% wenigstens einer Verbindung der Formel (I) nach Anspruch 1 oder 2 enthält.

9. Energetische Zusammensetzung nach Anspruch 6, **dadurch gekennzeichnet, daß** sie höchstens 80 Masse-% wenigstens einer Verbindung der Formel (I) nach Anspruch 1 oder 2 in einem Bindemittel enthält

10. Energetische Zusammensetzung nach einem der Ansprüche 6 bis 9, **dadurch gekennzeichnet, daß** sie aus einem schmelzgegossenen Explosivstoff besteht, der wenigstens eine Verbindung der Formel (I) nach Anspruch 1 oder 2 enthält.

11. Energetische Zusammensetzung nach Anspruch 10, **dadurch gekennzeichnet, daß** sie wenigstens ein weiteres energetisches Molekül enthält.

12. Energetische Zusammensetzung nach Anspruch 11, **dadurch gekennzeichnet, daß** sie wenigstens ein weiteres energetisches Molekül in einem Gehalt im Bereich zwischen 10 und 80 Masse-% enthält.
